# EUROPÄISCHE PATENTSCHRIFT

(11) **EP 0 986 995 B1**
(45) Veröffentlichungstag und Bekanntmachung des Hinweises auf die Patenterteilung: **01.10.2003**
(21) Anmeldenummer: 99810741.1
(22) Anmeldetag: 18.08.1999
(51) Int. Cl.: A61F 2/46, B01F 13/00

(54) **Umfüllvorrichtung für Knochenzement**
Transfer device for bone cement
Dispositif de transvasement pour ciment à os

(30) Priorität: 16.09.1998 EP 98810925
(43) Veröffentlichungstag der Anmeldung: 22.03.2000
(73) Patentinhaber: Centerpulse Orthopedics Ltd., 6340 Baar (CH)
(72) Erfinder: Heller, Mathias, 8404 Winterthur (CH); Suarez, Fernando, 8048 Zürich (CH)
(74) Vertreter: Manitz, Finsterwald & Partner Gbr

(56) Entgegenhaltungen:
- EP-A- 0 170 120
- EP-A- 0 380 867
- EP-A- 0 603 871
- EP-A- 0 747 114
- WO-A-87/05492
- WO-A-90/13264
- DE-A- 2 801 706
- DE-A- 3 717 134

## Beschreibung

Die Erfindung handelt von einer Umfüllvorrichtung für Knochenzement mit einem zylindrischen, nach oben offenen Mischbehälter und einem darin einsetzbaren Umfüllkolben, der abnehmbar mit der Hülse einer Zementspritze verbunden ist und eine Öffnung aufweist, durch welche der gemischte Knochenzement durch Niederdrücken des Umfüllkolbens in die Hülse umfüllbar ist.

Eine derartige Umfüllvorrichtung ist in der EP-A-0 261 182 ausführlich beschrieben. Diese hat jedoch den Nachteil, dass ein Umfüllkolben, der mit flüssigem Knochenzement benetzt ist zunächst aus einem Mischbehälter herausgezogen werden muss und von der eigentlichen Spritze losgelöst werden muss, um einen Ausstosskolben entsprechend dem Innenquerschnitt der Spritze aufzusetzen. Dabei sollte weder flüssiger Knochenzement zurückgesogen werden, noch verschüttet werden, noch an die Umgebung verteilt werden.

Aufgabe der Erfindung ist es, diese Situation zu verbessern. Diese Aufgabe wird mit den Kennzeichen vom unabhängigen Anspruch 1 dadurch gelöst, dass in der Hülse ein Ausstosskolben eingesetzt ist, der im Bereich der Öffnung für den durchfliessenden Zement einen Durchbruch aufweist, welcher nach dem Abnehmen der Zementspritze mit einem Stopfen verschliessbar ist. Dies hat den Vorteil, dass die eigentliche auf einen grossen Umfang verteilte und für das Auspressen dimensionierte dynamische Dichtung bereits installiert ist, wenn der Knochenzement in die Spritze gelangt und dass der kleinere verbleibende Durchbruch nur noch statisch gedichtet werden muss. Ausserdem ist der Querschnitt des Durchbruchs derart an die Viskosität eines bevorzugten Knochenzementes anpassbar, dass ein Rückfliessen weitgehend verhindert wird. Weitere Verbesserungen ergeben sich durch die abhängigen Ansprüche 2 bis 12. Schon ein Durchbruch mit einer kleinsten Durchtrittsfläche von weniger als 50 Prozent des inneren Querschnitts der Spritzenhülse bringt wesentliche Verbesserungen. Bei einem relativ dünnflüssigen Knochenzement hat sich eine Durchtrittsfläche von zwischen 25 und 5 Prozent des inneren Querschnitts der Spritzenhülse als vorteilhaft erwiesen. Bei so kleinen Durchbrüchen des Ausstosskolbens kann das Ende einer Kolbenstange einer Auspresspistole mit einem Stopfen versehen werden, der die statische Dichtung beim Auspressen vom Knochenzement übernimmt, weil die Druckkraft für den Auspresskolben auf einer wesentlich kleineren Fläche als der Kolbenfläche übertragen wird. Eine stirnseitige und eventuell elastische Schulter, welche sich der Neigung der Kolbenfläche vom Ausstosskolben anpassen kann genügt als statische Dichtung und hat den Vorteil, dass ausgehärteter Knochenzement nach Gebrauch einfach ablösbar ist.

Ein weiterer Vorteil ergibt sich, wenn die Öffnung des Umfüllkolbens als Düse ausgebildet ist, die in den Durchbruch des Ausstosskolbens hineinragt. Die eigentliche Restriktion des Knochenzements erfolgt dann in der Düse und es treten während dem Umfüllen nur noch geringe Mitnahmekräfte an Durchbruch und Ausstosskolben auf. Wenn die Mündung der Düse auf ihrer Aussenseite an dem Ausstosskolben anliegt, wird verhindert, dass die Unterseite vom Ausstosskolben von Knochenzement benetzt wird. Beim Ablösen von Spritzenhülse und Ausstosskolben wird, dann nur noch ein Faden von viskosem Knochenzement zwischen der Düse und dem Durchbruch gezogen. Eine leichte Vorpressung zwischen Düse und Ausstosskolben lässt sich erreichen, wenn der Ausstosskolben in einer unteren Position in der Hülse fixierbar ist, beispielsweise durch eine kraftund formschlüssige Schnappverbindung. Ein in der zylindrischen Innenfläche der Hülse, umlaufender Wulst, der sich erst bei einer grösseren Axialkraft am Ausstosskolben radial nach aussen verdrängen lässt, stellt eine solche Schnappverbindung dar.

Ein weiterer Vorteil ergibt sich, wenn der Umfüllkolben in seiner unteren Position so blockiert wird, dass die mit Zement gefüllte Hülse und der teilweise abschliessende Ausstosskolben in dieser untersten Stellung vom Umfüllkolben gelöst werden können. Wenn am Umfüllkolben ein zusätzlicher Griffteil angebracht ist, der in der untersten Position noch greifbar ist, dann kann eine Verbindung zwischen Hülse und Umfüllkolben in der tiefsten Stellung des Umfüllkolbens von Hand gelöst werden. Bei einem Drehverschluss wie bei einem Bajonettverschluss genügt es, den Griffteil und die Hülse gegeneinander zu drehen. Für einen Drehverschluss kann statt einem Griffteil in der untersten Position zwischen Umfüllkolben und Mischbehälter durch Vor- und Rücksprünge eine Drehsicherung angebracht werden, die ein zum Lösen notwendiges Drehmoment vom Mischbehälter auf den Umfüllkolben überträgt. Da der Umfüllkolben mit diesen Massnahmen nicht mehr herausgezogen werden muss, um die Hülse zu lösen, besteht auch nicht die Gefahr, dass Knochenzement aus der Spritze in den Mischbehälter zurückgesogen wird.

Ein Griffteil hat den Vorteil, dass relativ grosse Kräfte auf den Umfüllkolben zum Umfüllen des Knochenzements übertragbar sind, ohne dass dadurch eine Belastung der Verbindung zwischen Hülse und Umfüllkolben entsteht. Ausserdem kann der Querschnitt von Durchbruch und Düse kleiner gewählt werden, wenn grosse Kräfte auf den Umfüllkolben übertragen werden können.

Von der Handhabung her ist es günstig, die gleiche Art von Verschluss beispielsweise einen Bajonettverschluss zwischen Hülse und Umfüllkolben sowie zwischen Hülse und Auspresspistole vorzusehen. Die Hülse mit dem Knochenzement bleibt ohne Griffwechsel nach dem Ablösen vom Umfüllkolben in der gleichen Hand und kann mit einer umgekehrten Bewegung auf die Auspresspistole aufgesetzt werden.

Ein weiterer funktioneller Vorteil entsteht, wenn die Hülse einen konischen Übergang zu einem Verteilrohr besitzt und wenn sich dieser Konus im Ausstosskolben und in dem Durchbruch als Düse wiederholt. Mit dieser Massnahme wird der Ausflusswiderstand zum Verteilrohr verringert und es wird das vollständige Volumen bis in das Verteilrohr ausgepresst. Ausserdem wird eine stabile Führung des Ausstosskolbens erreicht, wenn der Kraftangriff der Kolbenstange praktisch oberhalb von der Kolbenfläche und oberhalb von der Dichtfläche zwischen Ausstosskolben und Hülse erfolgt. Es genügt eine kurze Führungsfläche vom Ausstosskolben in der Hülse, die mit der Dichtfläche zusammenfallen kann, da der Ausstosskolben mit dieser Aufhängung an der Kolbenstange keine Tendenz zum Verkanten zeigt.

Aus praktischen Gründen kann der Mischbehälter als Transportbehälter für eine der Komponenten vom Knochenzement ausgeführt sein, sodass ein ohnehin als Wegwerfteil konzipiertes Teil eine Mehrfachfunktion erfüllt. Eine pulverförmige Komponente des Knochenzements kann für den Transport mit einem Deckel oder einer Abreissfolie im Mischbehälter eingeschlossen sein. Nach dem Entfernen von Deckel oder Abreissfolie können weitere Komponenten hinzugegeben und von Hand mit einem Spatel vermischt werden. Anschliessend wird der Umfüllkolben aufgesetzt, auf welchem, die Spritzenhülse und der Ausstosskolben bereits vormontiert mitgeliefert wurden. Das Bedienungspersonal muss nach dem Mischen lediglich den Umfüllkolben einführen und niederpressen, die Hülse vom Umfüllkolben lösen und auf der Auspresspistole aufsetzen und dies ohne klebende Knochenzementreste an den von aussen greifbaren Flächen aufzubringen.

Im folgenden wird die Erfindung anhand von einem Ausführungsbeispiel beschrieben. Es zeigen:
- Fig. 1: Schematisch eine Ansicht eines Umfüllkolbens mit einem Griffteil, welches Längsrippen zur Führung des eigentlichen Kolbens aufweist;
- Fig. 2: schematisch einen Längsschnitt durch einen Mischbehälter der vorgängig als Transportbehälter und nach dem Mischen als Zylinder für einen Umfüllkolben nach Fig. 1 verwendbar ist;
- Fig. 3: schematisch einen Längsschnitt durch eine Zementspritze, in deren Hülse ein Ausstosskolben mit einem mittleren Durchbruch vormontiert ist;
- Fig. 4: schematisch einen Längsschnitt durch einen Umfüllkolben von Fig. 1, in welchen mit einem Bajonettverschluss eine Anordnung gemäss Fig. 3 einsetzbar ist;
- Fig. 5: schematisch im Längsschnitt einen Umfüllkolben mit Zementspritze in seiner untersten Stellung im Mischbehälter; und
- Fig. 6: schematisch im Längsschnitt die Zementspritze von Fig. 5 nach dem Aufsetzen auf eine Auspresspistole.

In den Figuren ist eine Umfüllvorrichtung für Knochenzement mit einem zylindrischen, nach oben offenen Mischbehälter 1 und mit einem darin einsetzbaren Umfüllkolben 2, der abnehmbar mit der Hülse 4 einer Zementspritze 3 verbunden ist und eine Öffnung 5 aufweist, durch welche der gemischte Knochenzement 6 durch Niederdrücken des Umfüllkolbens 2 in die Hülse 4 umfüllbar ist. Dabei ist in der Hülse 4 ein Ausstosskolben 7 eingesetzt der im Bereich der Öffnung 5 für den durchfliessenden Zement einen Durchbruch 8 aufweist, welcher nach dem Abnehmen der Zementspritze 3 mit einem Stopfen verschliessbar ist.

Die Hinweiszeichen in den Figuren sind für gleiche Begriffe verwendet. Da es sich bei den Teilen wie Mischbehälter 1, Deckel 21, Umfüllkolben 2, Zementspritze 3 mit Hülse 4 und mit Ausstosskolben 7 um Wegwerfteile mit grossen Stückzahlen handelt, werden diese in der Regel aus Thermoplasten auf Spritzgussmaschinen hergestellt.

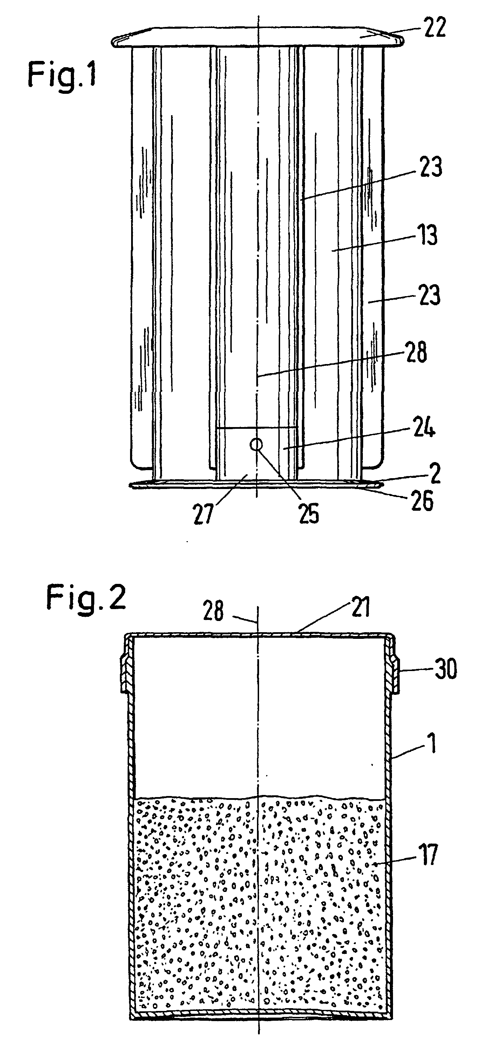

In Fig. 1 und 2 ist ein Umfüllkolben 2 mit einem zugehörigen Mischbehälter 1 gezeigt. Der eigentliche Kolben mit Kolbenfläche 26 ist dünnwandig ausgeführt und mit einem Griffteil 13 versehen, welches aus einem Rohrstück besteht, das wesentlich höher als der Mischbehälter 1 ist und in einem seitlich überstehenden Ring 22 endet, an welchem der Umfüllkolben von Hand bis zum Boden des Mischbehälters 1 niedergedrückt werden kann. Parallel zur Längsachse 28 sind am Rohrstück Längsrippen 23 über den Umfang verteilt, die als Führungen eine Schrägstellung der Kolbenfläche 26 und ihrer Dichtkante im Mischbehälter verhindern. Im unteren Teil des Rohrstücks sind zwei um 180° versetzte Fenster 24 angebracht, um auf einem weiter innenliegenden Ring 27 an seitlich entformbaren Werkzeugstempeln jeweils einen Zapfen 25 für einen Bajonettverschluss auszuspritzen.

Der Mischbehälter 1 ist als Transportbehälter gezeigt, welcher mit einem Deckel 21 über eine Schnappverbindung 30 verschlossen ist, um pulverförmige Anteile 17 vom Knochenzement aufzubewahren und zu transportieren. Zur Herstellung vom eigentlichen flüssigen Knochenzement wird der Deckel 21 seitlich aufgerissen und entfernt. Anschliessend wird eine Flüssigkomponente aus einem separaten Gefäss der Pulverkomponente 17 zugegeben und das ganze während einer vorgegebenen Reaktionszeit verrührt und möglichst gleichmässig gemischt. Der so entstandene und für eine begrenzte Zeit fliessfähige Knochenzement 6 muss nun in eine Zementspritze 3 umgefüllt werden.

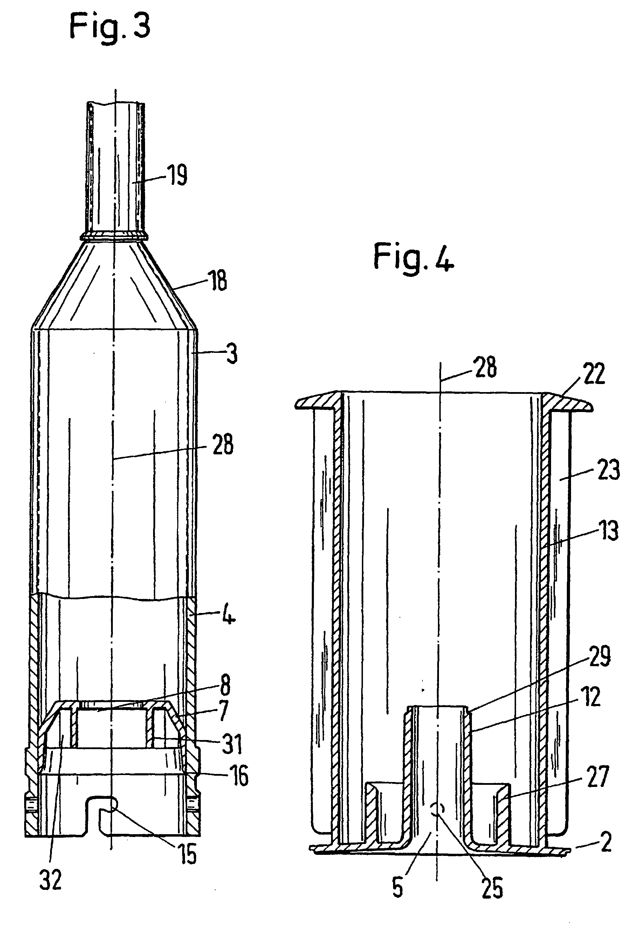

In Fig. 3 ist die eigentliche Zementspritze 3 gezeigt, die eine zylindrische Hülse 4 mit einem konischen Übergang 18 und ein Verteilrohr 19 aufweist. Der untere Rand der Hülse 4 ist nach aussen verstärkt und weist vier Aussparungen für einen Bajonettverschluss 15 auf. In einer unteren Position 16 ist mittels einer kleinen Änderung (nicht gezeigt) des Innendurchmessers der Hülse 4 ein Ausstosskolben 7 fixiert, dessen Kolbenfläche der Neigung vom konischen Übergang 18 angepasst ist. Der Ausstosskolben 7 besitzt im Zentrum einen Durchbruch 8, der den Aussenabmessungen einer Düse 12 (Fig. 4) angepasst ist. Durch einen Stützring 31 und radiale Rippen 32 erhält der Auspresskolben 7 eine hohe Steifigkeit gegen grosse Auspressdrücke. Die Fixierung des Auspresskolbens 7 in der unteren Position 16 ist so dimensioniert, dass er sich erst bei einer grösseren axialen Kraft nach oben bewegen kann.

In Fig. 4 sind die Elemente sichtbar, die auf die Hülse 4 und auf den Ausstosskolben 7 der Zementspritze 3 angepasst sind. Ein am Kolbenboden angeformter Führungsring 27 entspricht mit seinem Aussendurchmesser dem Innendurchmesser der Hülse 4 und besitzt zwei Zapfen 25, die zusammen mit den Aussparungen am unteren Ende der Hülse 4 einen Bajonettverschluss 15 bilden, welcher die axiale Lage zwischen Hülse 4 und Umfüllkolben 2 festlegt. Entsprechend dieser Lage und der unteren Position 16 des Ausstosskolbens 7 ist die Öffnung 5 des Umfüllkolbens 2 zu einer Düse 12 ausgeformt, die in den Ausstosskolben 7 hineinragt und die mit dem Betätigen des Bajonettverschlusses 15 an dem Ausstosskolben 7 mit einer Schulter 29 anliegt. Die Zementspritze 3 mit dem Ausstosskolben 7 und der Umfüllkolben 2 werden vor dem Einbringen des Umfüllkolbens 2 in den Mischbehälter 1 zusammengesetzt und über den Bajonettverschluss 15 gegeneinander verriegelt.

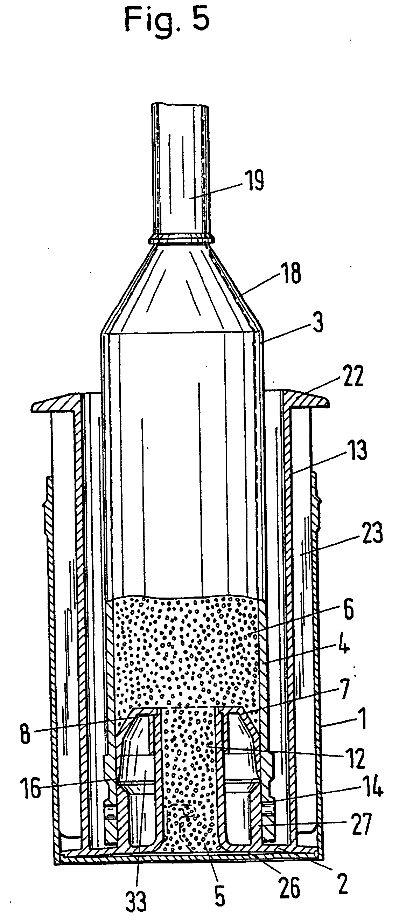

Die verriegelte Einheit von Umfüllkolben 2 und Zementspritze 3 wird am Griffteil 13 gefasst und von oben in einen Mischbecher 1 mit frischem Knochenzement 6 eingeschoben. Beim Auftreffen auf den Knochenzement 6, muss dieser durch die Mündung 12 hindurch in die Zementspritze 3 verdrängt werden. Entsprechend dem Durchmesser der Mündung 12 und der momentanen Viskosität des Knochenzements muss eine entsprechende Axialkraft am Ring 22 aufgebracht werden bis der Umfüllkolben 2, wie in Fig. 5 gezeigt, den Boden vom Mischbehälter 1 erreicht hat. In Fig. 5 durchstösst die Düse 12 den Auspresskolben 7 vollständig, sodass sich der in die Hülse 4 einfliessende Knochenzement 6 unter Schwerkraft gleichmässig in der Hülse 4 verteilt und diese langsam füllt, wobei die darüberstehende Luft durch das Verteilrohr 19 verdrängt wird.

Mit dem Erreichen des Bodens ist bis auf eine Restmenge in der Düse 12 der Knochenzement 6 vollständig in die Zementspritze 3 umgefüllt worden und reicht bis in das Verteilrohr 19 hinein. Bei einem sehr dünnflüssigen Knochenzement 6 kann nach dem Umfüllen eine (hier nicht gezeigte) Verschlusskappe auf dem Verteilrohr 19 angebracht werden, um ein Nachfliessen von Knochenzement bei der Trennung des Bajonettverschlusses 15 zwischen Umfüllkolben 2 und Zementspritze 3 zu verhindern. Unterhalb vom Auspresskolben 7 besteht ein Hohlraum 33, der so gut belüftet ist, dass beim Lösen des Bajonettverschlusses 15 und beim Abziehen der Zementspritze 3 an der Trennstelle zwischen dem Durchbruch 8 und der Schulter 29 Luft für eine Einschnürung und Trennung des Knochenzements 6 im Durchbruch 8 nachfliessen kann. Zum Lösen des Bajonettverschlusses 15 wird die Hülse 3 in ihrem oberen Bereich mit einer Hand gefasst, während mit der anderen Hand eine entsprechende Gegenkraft am Griffteil 13, 22 aufgebracht wird.

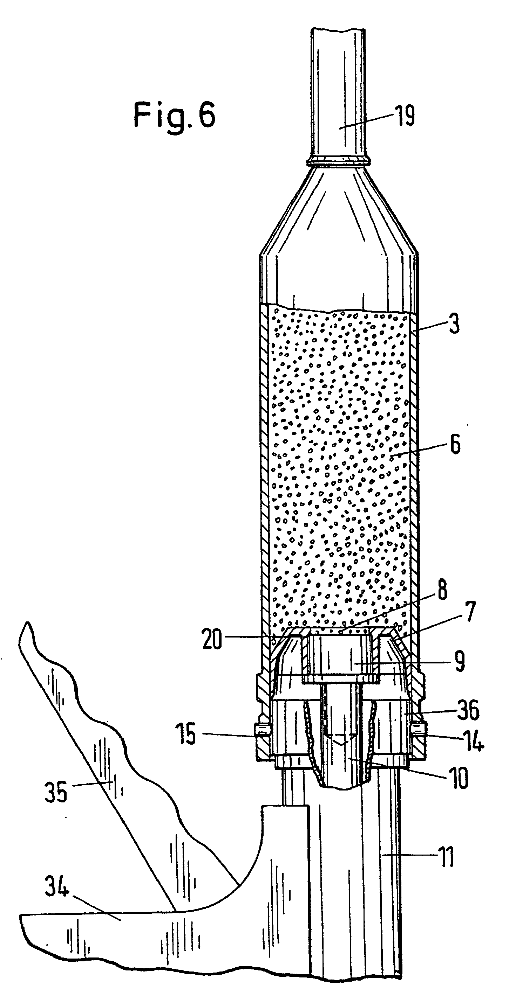

Die abgezogene Zementspritze 3 wird wie in Fig. 6 gezeigt auf eine Auspresspistole 11 aufgesteckt. Letztere besitzt eine ausfahrbare Kolbenstange 10, auf die ein Stopfen 9 aufgesteckt wurde, um mit dem Stopfen 9 den Durchbruch 8 abzudichten 20 und Ausstosskräfte auf den Ausstosskolben 7 zu übertragen. Die Kolbenstange 10 erfährt durch einen Abzughebel 35, der gegen einen Pistolengriff 34 bewegt wird, eine Vorschubbewegung, die stark untersetzt ist, um eine grosse Ausstosskraft zu erzeugen. Zur Übertragung einer grösseren Ausstosskraft greifen an allen vier Aussparungen der Hülse 4 Stifte ein, um den Bajonettverschluss 15 zu bilden. Die grosse Ausstosskraft ist notwendig, um die Querschnittsverringerung auf den Durchmesser des Verteilrohrs 19 und die Reibung an Verteilrohr 19 und Hülse 4 zu überwinden. Die Stifte sind in einem Anschlussstück 36 abgestützt, welches wie der Führungsring 27 im Umfüllkolben 2 auf den Innendurchmesser der Hülse 4 angepasst ist, um letzterer eine Ausrichtung in der Richtung der Vorschubbewegung der Kolbenstange 10 zu geben.

## Patentansprüche

1. Umfüllvorrichtung für Knochenzement mit einem zylindrischen, nach oben offenen Mischbehälter (1) und einem darin einsetzbaren Umfüllkolben (2), der abnehmbar mit der Hülse (4) einer Zementspritze (3) verbunden ist und eine Öffnung (5) aufweist, durch welche der gemischte Knochenzement (6) durch Niederdrücken des Umfüllkolbens (2) in die Hülse (4) umfüllbar ist, **dadurch gekennzeichnet, dass** in der Hülse (4) ein Ausstosskolben (7) eingesetzt ist, der im Bereich der Öffnung (5) für den durchfliessenden Zement einen Durchbruch (8) aufweist, welcher nach dem Abnehmen der Zementspritze (3) mit einem Stopfen (9) verschliessbar ist.

2. Umfüllvorrichtung nach Anspruch 1, **dadurch gekennzeichnet, dass** die kleinste Durchtrittsfläche im Durchbruch (8) weniger als 50 Prozent der inneren Querschnittsfläche der Hülse (4) entspricht.

3. Umfüllvorrichtung nach Anspruch 2, **dadurch gekennzeichnet, dass** die kleinste Durchtrittsfläche im Durchbruch (8) zwischen 25 und 5 Prozent der inneren Querschnittsfläche der Hülse (4) entspricht.

4. Umfüllvorrichtung nach Anspruch 1 bis 3, **dadurch gekennzeichnet, dass** der Stopfen (9) durch ein Ende einer Kolbenstange (10) gebildet ist als Bestandteil einer Auspresspistole (11), welche lösbar mit der Hülse (4) verbindbar ist.

5. Umfüllvorrichtung nach Anspruch 1 bis 4, **dadurch gekennzeichnet, dass** die Öffnung (5) als eine Düse (12) ausgeformt ist, die in den Durchbruch (8) des Ausstosskolbens (7) hineinragt.

6. Umfüllvorrichtung nach einem der Ansprüche 1 bis 5, **dadurch gekennzeichnet, dass** der Ausstosskolben (7) in einer unteren Position (16) in der Hülse (4) fixierbar ist.

7. Umfüllvorrichtung nach Anspruch 1 bis 6, **dadurch gekennzeichnet, dass** Hülse (4) und Umfüllkolben (2) durch einen Drehverschluss (14) beispielsweise durch einen Bajonettverschluss (15) verbindbar sind.

8. Umfüllvorrichtung nach Anspruch 1 bis 7, **dadurch gekennzeichnet, dass** der Umfüllkolben (2) einen Griffteil (13) aufweist, welcher ein Bewegen des Umfüllkolbens (2) von Hand ermöglicht.

9. Umfüllvorrichtung nach Anspruch 4 bis 8, **dadurch gekennzeichnet, dass** die Hülse (4) mit der gleichen Art von Verschluss sowohl mit dem Ausstosskolben (7) als auch mit der Auspresspistole (11) verbindbar ist.

10. Umfüllvorrichtung nach Anspruch 7, **dadurch gekennzeichnet, dass** der Umfüllkolben (2) mindestens in seiner untersten Position im Mischbehälter (1) gegen Drehen blockiert ist.

11. Umfüllvorrichtung nach Anspruch 1 bis 10, **dadurch gekennzeichnet, dass** der Mischbehälter (1) als verschliessbarer Transportbehälter für mindestens eine der Komponenten (17) eines zu mischenden Knochenzementes (6) ausgeführt ist.

12. Umfüllvorrichtung nach Anspruch 11, **dadurch gekennzeichnet, dass** der Mischbehälter (1) für den Transport von Komponenten von Knochenzement mit einem Deckel (21) oder mit einer Folie verschliessbar ist.

13. Umfüllvorrichtung nach Anspruch 8, **dadurch gekennzeichnet, dass** der Griffteil (13) mit Längsrippen (23) versehen ist, die in Längsrichtung am Mischbehälter (1) anliegen, um dem Umfüllkolben (2) eine Führung zu geben.

## Claims

1. Transfer filling apparatus for bone cement with a cylindrical, upwardly open mixing container (1) and a transfer filling piston (2) which can be inserted therein, which is removably connected to the sleeve (4) of a cement injector (3) and which has an opening (5) through which the mixed bone cement (6) can be transferred into the sleeve (4) through a pressing down of the transfer filling piston (2), **characterised in that** an ejection piston (7) is inserted in the sleeve (4) and has an aperture (8) in the region of the opening (5) for the through-flowing cement which can be closed by a plug (9) after the removal of the cement injector (3).

2. Transfer filling apparatus in accordance with claim 1 **characterised in that** the smallest passage area in the aperture (8) corresponds to less than 50 percent of the inner cross-sectional area of the sleeve (4).

3. Transfer filling apparatus in accordance with claim 2 **characterised in that** the smallest passage area in the aperture (8) corresponds to between 25 and 5 percent of the inner cross-sectional area of the sleeve (4).

4. Transfer filling apparatus in accordance with claims 1 to 3 **characterised in that** the plug (9) is formed by an end of a piston rod (10) as a constituent of an ejector pistol (11) which can be releasably connected to the sleeve (4).

5. Transfer filling apparatus in accordance with any one of claims 1 to 4 **characterised in that** the opening (5) is formed as a nozzle (12) which protrudes into the aperture (8) of the ejection piston (7).

6. Transfer filling apparatus in accordance with any one of claims 1 to 5 **characterised in that** the ejection piston (7) can be fixed in a lower position (16) in the sleeve (4).

7. Transfer filling apparatus in accordance with claims 1 to 6 **characterised in that** the sleeve (4) and the transfer filling piston (2) are connectable through a rotational lock (14), for example through a bayonet lock (15).

8. Transfer filling apparatus in accordance with claims 1 to 7 **characterised in that** the transfer filling piston (2) has a grip part (13) which enables a movement of the transfer filling piston (2) by hand.

9. Transfer filling apparatus in accordance with claims 4 to 8 **characterised in that** the sleeve (4) is connectable with the same kind of lock both to the ejection piston (7) and to the ejector pistol (11).

10. Transfer filling apparatus in accordance with claim 7 **characterised in that** the transfer filling piston (2) is blocked against rotation at least in its lowermost position in the mixing container (1).

11. Transfer filling apparatus in accordance with claim 1 to 10 **characterised in that** the mixing container (1) is made as a closable transport container for at least one of the components (17) of a bone cement (6) to be mixed.

12. Transfer filling apparatus in accordance with claim 11 **characterised in that** the mixing container (1) can be closed by a lid (21) or by a foil for the transport of components of bone cement.

13. Transfer filling apparatus in accordance with claim 8 **characterised in that** the grip part (13) is provided with longitudinal ribs (23) which lie in contact with the mixing container (1) in the longitudinal direction in order to provide a guide for the transfer filling piston (2).

## Revendications

1. Dispositif de transvasement pour du ciment à os avec un récipient de mélange cylindrique (1), ouvert vers le haut et un piston de transvasement (2) pouvant être placé dans celui-ci qui est relié amoviblement à la douille (4) d'une seringue à ciment (3) et qui présente une ouverture (5) par laquelle le ciment à os mélangé (6), en enfonçant le piston de transvasement (2), peut être transvasé dans la douille (4), **caractérisé en ce qu'**il est placé dans la douille (4) un piston d'éjection (7) qui présente au voisinage de l'ouverture (5) pour le ciment s'écoulant à travers celle-ci un perçage (8) qui peut être fermé, après le retrait de la seringue à ciment (3), avec un bouchon (9).

2. Dispositif de transvasement selon la revendication 1, **caractérisé en ce que** la face de passage la plus petite dans le perçage (8) correspond à moins que 50 pour cent de la face intérieure en section transversale de la douille (4).

3. Dispositif de transvasement selon la revendication 2, **caractérisé en ce que** la face de passage la plus petite dans le perçage (8) correspond à entre 25 et 5 pour cent de la face intérieure en section transversale de la douille (4).

4. Dispositif de transvasement selon la revendication 1 à 3, **caractérisé en ce que** le bouchon (9) est formé par une extrémité d'une tige de piston (10), en tant que composant d'un pistolet d'éjection (11), qui peut être relié amoviblement à la douille (4).

5. Dispositif de transvasement selon la revendication 1 à 4, **caractérisé en ce que** l'ouverture (5) est réalisée comme une buse (12) qui fait saillie dans le perçage (8) du piston d'éjection (7).

6. Dispositif de transvasement selon l'une des revendications 1 à 5, **caractérisé en ce que** le piston d'éjection (7) peut être fixé dans une position inférieure (16) dans la douille (4).

7. Dispositif de transvasement selon la revendication 1 à 6, **caractérisé en ce que** la douille (4) et le piston de transvasement (2) peuvent être reliés par une fermeture tournante (14), par exemple par une fermeture à baïonnette (15).

8. Dispositif de transvasement selon la revendication 1 à 7, **caractérisé en ce que** le piston de transvasement (2) présente une pièce de préhension (13) qui permet un déplacement du piston de transvasement (2) à la main.

9. Dispositif de transvasement selon la revendication 4 à 8, **caractérisé en ce que** la douille (4) peut être reliée avec le même type de fermeture à la fois au piston d'éjection (7) et aussi au pistolet d'éjection (11).

10. Dispositif de transvasement selon la revendication 7, **caractérisé en ce que** le piston de transvasement (2) est bloqué au moins dans sa position la plus inférieure dans le récipient de mélange (1) à l'encontre d'une rotation.

11. Dispositif de transvasement selon la revendication 1 à 10, **caractérisé en ce que** le récipient de mélange (1) est réalisé comme un récipient de transport pouvant être fermé pour au moins l'un des composants (17) d'un ciment à os (6) à mélanger.

12. Dispositif de transvasement selon la revendication 11, **caractérisé en ce que** le récipient de mélange (1), pour le transport de composants du ciment à os, peut être fermé avec un couvercle (21) ou avec une feuille.

13. Dispositif de transvasement selon la revendication 8, **caractérisé en ce que** la pièce de préhension (13) présente des nervures longitudinales (23) qui s'appliquent dans la direction longitudinale au récipient de mélange (1) pour conférer au piston de transvasement (2) un guidage.
